# EUROPÄISCHE PATENTSCHRIFT

(11) **EP 3 600 562 B1**
(45) Veröffentlichungstag und Bekanntmachung des Hinweises auf die Patenterteilung: **07.06.2023**
(21) Anmeldenummer: 18724782.0
(22) Anmeldetag: 20.03.2018
(51) Int. Cl.: A61Q 11/00, A61K 8/92

(54) **ZAHNPFLEGEMITTEL**
DENTAL CARE AGENT
PRODUIT DENTIFRICE

(30) Priorität: 20.03.2017 DE 202017101612 U; 20.03.2017 DE 202017101613 U
(43) Veröffentlichungstag der Anmeldung: 05.02.2020
(73) Patentinhaber: Fritsch, Tilman, 83457 Bayerisch Gmain (DE)
(72) Erfinder: Fritsch, Tilman, 83457 Bayerisch Gmain (DE)
(74) Vertreter: Patentanwälte und Rechtsanwalt Weiß, Arat & Partner mbB
(86) Internationale Anmeldenummer: PCT/EP2018/057002
(87) Internationale Veröffentlichungsnummer: WO 2018/172345

(56) Entgegenhaltungen:
- DE-A1- 19 649 641
- DE-U1- 20 101 649
- DATABASE GNPD [Online] MINTEL; Oktober 2016 (2016-10), "Oil Pulling Cure", XP002782574, Database accession no. 4366483
- DATABASE GNPD [Online] MINTEL; April 2015 (2015-04), "Organic Toothpaste with Medicinal Clay and Propolis", XP002782575, Database accession no. 3079839

## Beschreibung

### Technisches Gebiet

Die Erfindung betrifft ein Zahnpflegemittel nach dem Oberbegriff des Anspruchs 1.

### Stand der Technik

In der DE 196 49 641 A1 ein Zahn- und Mundpflegemittel mit 20-60 Gew.-%, Kochsalz, 20-60% Gew.-% Sonnenblumenöl sowie Füllstoffen und weiteren Inhaltsstoffen.

Weiter wird auf die DE 201 01 649 U1 hingewiesen, welche ein Mundspülöl unter anderem aus 80-99,9 Gew.-% aus pflanzlichem Öl besteht.

Zahncremes und andere Zahnpflegemittel sind bereits in vielfältiger Form und Ausgestaltung bekannt und gebräuchlich.

Zahncremes sind bekannt und gebräuchlich um die Wirkung der mechanischen Zahnreinigung, üblicherweise mittels einer Zahnbürste, zu erhöhen.

Bakterien im Mundraum und insbesondere auf den Zähnen sind für Zahnfleischentzündung, Zahnstein- und Kariesbildung sowie schlechten Atem zumindest mitverantwortlich. Um die Anzahl der Bakterien im Mundraum und insbesondere auf den Zähnen zu verringern und/oder deren Wachstum zu hemmen, werden bekannten Zahncremes Triclosan, Zinksalze oder andere bakterizide Stoffe beigemischt. Diese Stoffe haben oft unerwünschte Nebenwirkungen, sind teilweise schwer verträglich und es besteht die Gefahr von Resistenzbildungen.

Bekannte Zahncremes enthalten oft aggressive Putzkörper, welche den Zahnschmelz schädigen.

Oftmals werden Zahncremes Konservierungsmittel wie beispielsweise Hydroxybenzoesäureester oder dergleichen zugesetzt. Ferner enthalten bekannte Zahncremes oftmals Zusatzstoffe. Nachteilig hieran ist, dass einige Menschen auf Konservierungsmittel und andere üblicherweise in Zahncremes eingesetzte Zusatzstoffe reagieren, mit Allergien und/oder Unverträglichkeiten.

Vielen Zahncremes wird seit langem Fluorid zur Kariesprophylaxe beigesetzt.

Zur Reduzierung der Oberflächenspannung werden Zahncremes oftmals Detergentien und/oder Entspannungsmittel beigemengt.

Mundspülungen, teilweise auch als Mundwasser oder Mundspül-Lösung bezeichnet, sind in vielfältiger Form und Ausgestaltung bekannt und gebräuchlich.

Mundspülungen werden zur Zahnerkrankungs-Prophylaxe angewandt. Sie bestehen meist aus einer Flüssigkeit, welche Bakterien und Keime im Mundraum dezimieren soll.

Bekannte Mundspülungen sind in der Regel ein Alkohol-Wasser-Gemisch und enthalten meist weitere Inhaltsstoffe. Der Alkohol, beispielsweise Ethanol oder Propandiol, kann hierbei der Desinfektion und/oder der Konservierung dienen. Ferner lösen sich viele Stoffe, welche häufig in Mundspülungen verwendet werden, deutlich besser in einem Alkohol-Wasser-Gemisch als in reinem Wasser, so dass der Alkohol auch als Lösungsvermittler dienen kann.

So werden bekannten Mundspülungen beispielsweise antiseptische Wirkstoffe wie Chlorhexidin beigesetzt, welche sich teilweise deutlich besser in Alkohol-Wasser-Gemischen lösen als in reinem Wasser.

Ferner enthalten einige bekannte Mundspülungen Fluoride, welche der Härtung des Zahnschmelzes dienen sollen.

Bekannte Mundspülungen weisen jedoch zahlreiche Nachteile auf.

Einerseits wird ein Teil des in der Mundspülung enthaltenen Alkohols, insbesondere, wenn es sich um Ethanol handelt, bereits beim morgendlichen Gurgeln über die Mundschleimhaut in den Organismus aufgenommen. Der Blutalkoholgehalt steigt, was zu sämtlichen Folgen führt, welche auch beim Genuss alkoholischer Getränke auftreten, beispielsweise vermindertem Reaktionsvermögen und negativen Auswirkungen auf die Gesundheit.

Weiterhin wirken zahlreiche Alkohole zytotoxisch auf die Zellen der Mundhöhle, was wiederum zu verminderter Widerstandsfähigkeit und Entzündungsneigung dieser Zellen führt. Eigentlich versucht man mit umfassender Mundhygiene, beispielsweise also mit dem Gebrauch von Mundspülungen, diesem Effekt gerade entgegenzutreten.

Insbesondere, wenn gleichzeitig fluoridhaltige Zahncreme verwendet wird, birgt die Anwendung fluoridhaltiger Mundspülungen die Gefahr der Entstehung einer Fluorose, also einer Erkrankung, welche durch übermäßige Fluoridzufuhr ausgelöst wird. Diese kann sich sowohl auf der Zahnoberfläche als auch systemisch manifestieren.

Antiseptische Wirkstoffe haben stets Nebenwirkungen, welche sich bei manchen Benutzern ebenfalls sehr negativ auf die körperliche Gesundheit, insbesondere die Gesundheit des Mundraums, auswirken können.

Auch weitere Inhaltsstoffe, welche in bekannten Mundspülungen zum Einsatz kommen, können sich negativ auf die Gesundheit des Benutzers auswirken. Nur beispielhaft sei hier auf Konservierungsstoffe hingewiesen.

Hinzu kommt, dass ein Verschlucken bekannter Mundspülungen meist als gesundheitsschädlich eingestuft werden muss. Die vorstehend beschriebenen unerwünschten Wirkungen bekannter Mundspülungen stellen sich zwar schon beim bestimmungsgemäßen Gebrauch ein, gemäß dem die Mundspülung nach dem Spülen ausgespuckt wird. Wird die Mundspülung dennoch versehentlich verschluckt, so verstärken sich die beschriebenen negativen Wirkungen durch die Aufnahme der Mundspülung in den Verdauungstrakt entsprechend.

### Aufgabe der Erfindung

Die Aufgabe der vorliegenden Erfindung ist es, die Nachteile aus dem Stand der Technik zu überwinden. Es soll ein Zahnpflegemittel, insbesondere eine Zahncreme bereitgestellt werden, welches die positiven Eigenschaften bekannter Zahnpflegemittel erreicht, deren Nachteile jedoch weitest möglich beseitigt. Weiterhin soll ein Zahnpflegemittel bereitgestellt werden, welches zur Entgiftung des Körpers beiträgt und Beschädigungen der Zahnoberfläche möglichst vermeidet.

### Lösung der Aufgabe

Zur Lösung der Aufgabe führen die Merkmale nach dem Anspruch 1.

Vorteilhafte Ausgestaltungen sind in den Unteransprüchen beschrieben.

Bei dem Zahnpflegemittel gemäss der vorliegenden Erfindung kann es sich um eine Zahncreme handeln.

Das erfindungsgemässe Zahnpflegemittel enthält ein gesundheitsverträgliches Öl. Hierbei kann es sich beispielsweise um Sonnenblumenöl handeln. Es kann auch an Hanföl, Leinöl oder Sesamöl gedacht sein. Das Zahnpflegemittel enthält zwischen 25 und 50 Gew.-% des gesundheitsverträglichen Öls, beispielsweise des Sonnenblumenöls, des Hanföls, des Leinöls oder des Sesamöls enthalten. Vorzugsweise kann daran gedacht sein, dass das Zahnpflegemittel 30-35 Gew.-%, beispielsweise 33,2 Gew.-% des gesundheitsverträglichen Öls enthält.

Erfindungsgemäß besteht das Zahnpflegemittel aus einer Mischung aus zumindest zwei gesundheitsverträglichen Ölen, also eine Mischung bestehend aus zumindest zwei der vorgenannten Öle.

Gesundheitsverträgliche Öle, insbesondere Sonnenblumenöl, Hanföl, Leinöl und Sesamöl verfügen über sehr hohe Bindungseigenschaften, weshalb sie sich hervorragend zur Entgiftung des Körpers eignen. Giftstoffe wie beispielsweise Toxine, Metalle oder dergleichen werden vom Öl gebunden und aus dem Körper ausgeschieden, beispielsweise, wenn der Benutzer nach dem Zähneputzen ausspuckt oder den Mund ausspült.

Weiterhin kann darauf verzichtet werden, der erfindungsgemässen Zahncreme Entspannungsmittel oder Detergentien, beispielsweise chemische Detergentien, beizumengen, da Öle eine geringe Oberflächenspannung haben.

Das Zahnpflegemittel enthält Sole. Unter Sole wird hierbei erfindungsgemäß eine wässrige Salzlösung verstanden, welche zumindest 14g Salz pro kg Wasser enthält. Bei der Sole handelt es sich um eine Lösung aus Natriumchlorid in Wasser. Die Konzentration der Sole beträgt über 15 Gew.-%, kann im Einzelnen auch über 20 Gew.-% oder sogar über 25 Gew.-%, beispielsweise 26 Gew.-% oder 28 Gew.-% betragen.

Das Zahnpflegemittel kann zwischen 25 und 50 Gew.-% Sole enthalten. Vorzugsweise kann daran gedacht sein, dass das Zahnpflegemittel 30-35 Gew.-%, beispielsweise 33,2 Gew.-% Sole enthält.

Die Sole hat sowohl strukturierende als auch neutralisierende Eigenschaften. Sie kann also beispielsweise den pH-Wert im Mundraum erhöhen. Dies wirkt sich positiv aus, da ein niedriger pH-Wert in Verbindung mit mechanischer Beanspruchung der Zahnoberfläche durch die Zahnbürste eine Beschädigung der Zahnoberfläche bewirken kann.

Ferner senkt die Sole die Gefahr der Zahnsteinbildung.

Um den Geschmack des Zahnpflegemittels positiv zu beeinflussen, kann an eine Sole auf Hydrolatbasis gedacht sein. Hierbei werden Hydrolate, insbesondere geschmacklich relevante Hydrolate, beispielsweise Lemongras-, Zitronen-, Salbei- oder ähnliche Hydrolate, durch Salzzugabe zu einer Sole, insbesondere zu einer gesättigten Sole, verarbeitet. Bei dem Salz kann es sich um Kochsalz handeln.

Das Zahnpflegemittel kann zwischen 5 und 10 Gew.-% Kreide enthalten. Vorzugsweise kann daran gedacht sein, dass das Zahnpflegemittel 6 bis 7 Gew.-% beispielsweise 6,6 Gew.-% Kreide enthält. Bei der Kreide kann es sich um Kreidegestein oder um Kalkstein handeln, also beispielsweise um reines Calcit bzw. Calciumcarbonat. Weiterhin kann auch an Calciumsulfat und Magnesiumoxid gedacht sein.

Kreide ist im Vergleich zu anderen Putzkörpern sehr weich und greift den Zahnschmelz nicht an, der Zahnschmelz wird nicht abradiert. Ferner weist Kreide hervorragende Putzeigenschaften auf.

Das Zahnpflegemittel kann zwischen 15 und 50 Gew.-% Heilerde enthalten. Vorzugsweise kann daran gedacht sein, dass das Zahnpflegemittel 20 bis 30 Gew.-% beispielsweise 26,6 Gew.-% Heilerde enthält.

Unter Heilerde können hierbei eiszeitliche Lössablagerungen verstanden werden, welche beispielsweise in Pulverform bekannt sind. Diese können, teilweise oder im Wesentlichen, aus Aluminium-Silikaten bestehen. Ferner kann es sich um Löss-, Lehm-, Ton- oder Moorerden handeln. Heilerde kann ferner, teilweise oder im Wesentlichen, aus Tonmineralen wie beispielsweise Hectorite bestehen. Der Anteil der Hectorite in dem Zahnpflegemittel kann beispielsweise 25 bis 50 Gew.-% betragen.

Das Zahnpflegemittel kann weitere Putzkörper enthalten. Da das Zahnpflegemittel vorzugsweise kein Fluorid enthält, ist der Zusatz von natürlichen Putzkörpern, wie beispielsweise der oben erwähnten Kreide bzw. Calcit, möglich.

Ferner kann einerseits daran gedacht sein, dass Zahnpflegemittel nur eine Sorte eines Putzkörpers, beispielsweise ausschliesslich Kreide, enthält. Andererseits kann daran gedacht sein, dass das Zahnpflegemittel mehrere Sorten von Putzkörpern enthält.

Das Zahnpflegemittel kann zwischen 0,1 und 1 Gew.-% eines ätherischen Öls enthalten. Beispielsweise kann 0,4 Gew.-% eines ätherischen Öls enthalten sein. Das ätherische Öl kann dazu dienen, dem Zahnpflegemittel einen angenehmen Geschmack zu verleihen. Bei dem ätherischen Öl kann es sich um Nelkenminzen-Öl (bspw. Nelkenminze SL), Orange-Zimt-Öl oder Lemongras-Öl (bspw. Lemongras SL) handeln. Ferner kann auch an Thymian- oder NEEM-ÖI, welches aus dem Neem-Baum gewonnen wird, gedacht sein.

Gesundheitsverträgliche Öle und insbesondere ätherische Öle reduzieren die Anzahl der Bakterien im Mundraum und auf den Zähnen, ausserdem hemmen sie das Bakterienwachstum.

Das Zahnpflegemittel kann in einem Violettglas aufbewahrt und gelagert werden. Vorzugsweise handelt es sich hierbei um ein UV-beständiges Violettglas. Derartige Violettgläser schützen das Zahnpflegemittel vor äusseren Einwirkungen und enthalten kaum oder keinerlei Lösungsmittel, Weichmacher oder Kunststoffe.

Das Zahnpflegemittel enthält vorzugsweise keine Konservierungsstoffe. Ferner enthält das Zahnpflegemittel vorzugsweise keine Zusatzstoffe.

Vorzugsweise handelt es sich bei sämtlichen Inhaltsstoffen um natürliche Inhaltsstoffe, also um Stoffe natürlichen Ursprungs. Vorzugsweise handelt es sich, sofern möglich, um regionale Stoffe, die also nicht über weite Strecken transportiert werden müssen.

Ferner enthält das Zahnpflegemittel vorzugsweise kein Fluorid. Dank der Zusammensetzung des erfindungsgemässen Zahnpflegemittels kann auf den Einsatz von Fluorid verzichtet werden. Dadurch lassen sich sämtliche unerwünschten Wirkungen des typischerweise in Zahnpflegemitteln enthaltenen Fluorids vermeiden. So besteht beim Einsatz von Fluorid beispielsweise die Gefahr der Zahnfluorose, bei der sich die Zähne fleckig verfärben, was sowohl ein optisches als auch ein medizinisches Problem ist, da die Widerstandsfähigkeit des Zahnschmelzes reduziert wird. Auch chronische Schädigungen des Gesamtorganismus durch Fluorid, welches bei der Anwendung bekannter Zahnpflegemittel aufgenommen werden kann, können durch das vorzugsweise fluoridfreie erfindungsgemässe Zahnpflegemittel vermieden werden.

Das erfindungsgemässe Zahnpflegemittel, bei dem es sich vorzugsweise um eine Zahncreme handelt, lässt sich verwenden wie bekannte Zahnpflegemittel, insbesondere wie bekannte Zahncremes. Das erfindungsgemässe Zahnpflegemittel kann vor der Verwendung auch mit einem Holzspatel auf eine Zahnbürste aufgetragen werden.

Das Zahnpflegemittel enthält üblicherweise Wasser, hierbei kann an einen Gehalt von 10 bis 25 Gew.-% gedacht sein.

Ferner kann das Zahnpflegemittel, abhängig von der Zusammensetzung der beschriebenen, vorzugsweise natürlichen Inhaltsstoffe zwischen 5 und 10 Gew.-% Natriumchlorid und/oder zwischen 5 und 10 Gew.-% Calciumcarbonat enthalten.

Durch den geringen Eigengeschmack des Zahnpflegemittels kommt es zu keinen oder nur zu geringfügigen Geschmacksirritationen. Derartige Geschmacksirritationen treten bei bekannten Zahnpflegemitteln in deutlich größerem Umfang auf. Durch die geringen Geschmacksirritationen wird das retrograde Riechen, auch retronasale Aromawahrnehmung genannt, nicht gestört. Bei diesem Vorgang gelangen flüchtige Verbindungen aus dem Mundraum zu den olfaktorischen Rezeptoren in der Nase, was wiederum ausschlaggebend für eine der aufgenommenen Nahrung angemessene Verdauung ist.

Da das retrograde Riechen nicht gestört wird, kann das Zahnpflegemittel gemäss der vorliegenden Erfindung im Gegensatz zu bekannten Zahncremes unmittelbar nach dem Essen benutzt werden. Hierbei wird eine vorteilhafte Mundhygiene unmittelbar nach der Nahrungsaufnahme erreicht, ohne dass die Verdauung negativ beeinflusst wird.

Der vorstehend beschriebe Effekt in Bezug auf das retrograde Riechen wird erreicht, da das erfindungsgemässe Zahnpflegemittel auf zahlreiche Stoffe verzichtet, welche in bekannten Zahnpflegemitteln enthalten sind. Das Zahnpflegemittel kann also ausschliesslich aus zumindest einem der folgenden Stoffe bestehen: gesundheitsverträgliches Öl, insbesondere Sonnenblumenöl, Hanföl, Leinöl oder Sesamöl, ätherisches Öl, Sole, Kreide, Heilerde.

Das Zahnpflegemittel gemäss der vorliegenden Erfindung kann demnach ausschliesslich aus einem gesundheitsverträglichen Öl oder einer Mischung solcher Öle bestehen. Hierbei kommen sämtliche der beschriebenen Öle in Betracht.

Demnach kann das Zahnpflegemittel nach einem weiteren Ausführungsbeispiel der vorliegenden Erfindung ausschliesslich aus einem gesundheitsverträglichen Öl oder einer Mischung solcher Öle sowie aus zumindest einem ätherischen Öl bestehen. Hierbei kommen sämtliche der beschriebenen Öle sowie sämtliche der beschriebenen ätherischen Öle in Betracht.

Demnach kann das Zahnpflegemittel nach einem weiteren Ausführungsbeispiel der vorliegenden Erfindung ausschliesslich aus einem gesundheitsverträglichen Öl oder einer Mischung solcher Öle sowie aus zumindest einem ätherischen Öl und aus einer Sole bestehen. Hierbei kommen sämtliche der beschriebenen Öle sowie sämtliche der beschriebenen ätherischen Öle und Solen in Betracht.

Demnach kann das Zahnpflegemittel nach einem weiteren Ausführungsbeispiel der vorliegenden Erfindung ausschliesslich aus einem gesundheitsverträglichen Öl oder einer Mischung solcher Öle sowie zusätzlich aus zumindest einem ätherischen Öl und aus einer Sole und aus Kreide bestehen. Hierbei kommen sämtliche der beschriebenen Öle sowie sämtliche der beschriebenen ätherischen Öle und Solen und Kreide-Varianten in Betracht.

Demnach kann das Zahnpflegemittel nach einem Ausführungsbeispiel der vorliegenden Erfindung ausschliesslich aus einem gesundheitsverträglichen Öl oder einer Mischung solcher Öle sowie zusätzlich aus zumindest einem ätherischen Öl und aus einer Sole und aus Kreide und aus Heilerde bestehen. Hierbei kommen sämtliche der beschriebenen Öle sowie sämtliche der beschriebenen ätherischen Öle und Solen und Kreide-Varianten und Heilerde-Varianten in Betracht.

Die erfindungsgemässe Mundspülung enthält Sonnenblumenöl. Vorzugsweise enthält die Mundspülung zumindest 75 Gew.-% Sonnenblumenöl.

Die Mundspülung kann zwischen 90 und 95 Gew.-% Sonnenblumenöl enthalten, beispielsweise 93 Gew.-%.

Ferner kann die Mundspülung Schwarzkümmelöl enthalten. Die Mundspülung kann beispielsweise bis zu 10 Gew.-% Schwarzkümmelöl enthalten. Beispielsweise kann die Mundspülung 5 bis 10 Gew.-% Schwarzkümmelöl enthalten, 5 Gew.-% sind besonders bevorzugt.

Weiterhin kann die Mundspülung Salbeiöl enthalten. Die Mundspülung kann beispielsweise zwischen 1 und 5 Gew.-% Salbeiöl enthalten. Hierbei ist ein Gehalt von 2 Gew.-% Salbeiöl besonders bevorzugt.

Insbesondere ist an eine Mundspülung gedacht, welche 75 bis 94 Gew.-% Sonnenblumenöl, 5 bis 10 Gew.-% Schwarzkümmelöl und 1 bis 5 Gew.-% Salbeiöl enthält.

Die Anwendung der erfindungsgemässen Mundspülung erfolgt wie bei bekannten Mundspülungen durch Gurgeln. Vorzugsweise wird die erfindungsgemässe Mundspülung hierbei 5 bis 10 Minuten gegurgelt, um sämtliche Abschnitte des Mundraums ausreichend zu benetzen.

Die vorgenannten Öle sind vorzugsweise von höchster Qualität und stammen vorzugsweise aus kontrolliert biologischem Anbau.

Im Gegensatz zu bekannten Mundspülungen stabilisieren die vorgenannten Öle die Bildung einer physiologischen Mundflora. Bekannte Mundspülungen bringen diese Mundflora aus dem Gleichgewicht oder zerstören sie sogar.

Die vorgenannten Öle wirken entzündungshemmend. Sie dienen ferner der Vorbeugung von Parodontose und reduzieren die Anzahl unerwünschter Keime und Bakterien im Mundraum.

Die vorgenannten Öle haben ausserdem sehr hohe Bindungseigenschaften. Da sie Toxine, Metalle, Schadstoffe und dergleichen binden, können diese unerwünschten Stoffe nach dem Gurgeln mit der erfindungsgemässen Mundspülung durch Ausspucken schonend aus dem Körper entfernt werden.

Die Anwendung der erfindungsgemässen Mundspülung stärkt die Muskeln im Mundraum, pflegt das Zahnfleisch und beugt Entzündungen vor.

Vorzugsweise enthält die Mundspülung keine weiteren Inhaltsstoffe ausser den drei vorgenannten Ölen, sie kann daher zu Recht als Naturprodukt bezeichnet werden. Insbesondere enthält die Mundspülung gemäss der vorliegenden Erfindung keine Konservierungsstoffe und keinen Alkohol.

Ein Verschlucken der Mundspülung entspricht zwar nicht dem bestimmungsgemäss Gebrauch, fügt dem Organismus aber auch keinen Schaden zu. Beim versehentlichen Verschlucken werden lediglich die vorgenannten gut verträglichen Öle zusammen mit den eigentlich durch Ausspucken zu entfernenden unerwünschten Stoffen in den Magen-Darm-Trakt aufgenommen. Im Gegensatz zu bekannten Mundspülungen enthält die vorliegende Mundspülung selbst jedoch keine Inhaltsstoffe, welche sich negativ auf die Gesundheit auswirken.

Schwarzkümmelöl und Salbeiöl haben eine antifungale und antibakterielle Wirkung.

Die vorliegende Erfindung schlägt ferner ein Violettglas zur Lagerung und Aufbewahrung der Mundspülung vor. Vorzugsweise handelt es sich hierbei um ein UV-beständiges Violettglas. Derartige Violettgläser schützen die Mundspülung vor äusseren Einwirkungen und enthalten kaum oder keinerlei Lösungsmittel, Weichmacher oder Kunststoffe.

## Patentansprüche

1. Zahnpflegemittel zur Behandlung der Zähne, wobei das Zahnpflegemittel ein gesundheitsverträgliches Öl enthält, das Zahnpflegemittel zwischen 0,1 und 1 Gew.-% eines ätherischen Öls enthält, wobei das gesundheitsverträgliche Öl Sonnenblumenöl, Hanföl, Leinöl oder Sesamöl ist,
**dadurch gekennzeichnet, dass**
dass Zahnpflegemittel eine Mischung bestehend aus zumindest zwei gesundheitsverträglichen Ölen enthält und das Zahnpflegemittel 33,2 Gew.-% Sole enthält, wobei das Zahnpflegemittel zwischen 25 und 50 Gew.-% des gesundheitsverträglichen Öls enthält, wobei die Sole eine wässrige Salzlösung ist, welche zumindest 14g Salz pro kg Wasser enthält, wobei es sich bei der Sole um eine Lösung aus Natriumchlorid in Wasser handelt und die Konzentration der Sole über 15 Gew.-% beträgt.

2. Zahnpflegemittel nach einem der vorigen Ansprüche, **dadurch gekennzeichnet, dass** es sich bei der Sole um eine Sole auf Hydrolatbasis handelt.

3. Zahnpflegemittel nach einem der vorhergehenden Ansprüche, **dadurch gekennzeichnet, dass** das Zahnpflegemittel zwischen 5 und 10 Gew.-% Kreide enthält.

4. Zahnpflegemittel nach einem der vorhergehenden Ansprüche, **dadurch gekennzeichnet, dass** das Zahnpflegemittel zwischen 15 und 50 Gew.-% Heilerde enthält.

## Claims

1. Dentifrice for the treatment of teeth, wherein the dentifrice contains a health-compatible oil, the dentifrice contains between 0.1 and 1 wt.% of an essential oil, wherein the health-compatible oil is sunflower oil, hemp oil, linseed oil or sesame oil,
**characterized in that**
that dentifrice contains a mixture consisting of at least two health-compatible oils and the dentifrice contains 33.2 wt.% of brine, wherein the dentifrice contains between 25 and 50 wt.% of the health-compatible oil, wherein the brine is an aqueous salt solution containing at least 14g of salt per kg of water, wherein the brine is a solution of sodium chloride in water and the concentration of the brine being above 15 wt.%.

2. Dentifrice according to any one of the preceding claims, **characterized in that** the brine is a hydrolate-based brine.

3. Dentifrice according to one of the preceding claims, **characterized in that** the dentifrice contains between 5 and 10% by weight of chalk.

4. Dentifrice according to any one of the preceding claims, **characterized in that** the dentifrice contains between 15 and 50 wt.% of healing clay.

## Revendications

1. Agent de soins dentaires permettant de traiter des dents, dans lequel l'agent de soins dentaires contient une huile respectueuse de la santé, l'agent de soins dentaires contient entre 0,1 et 1 % en poids d'une huile essentielle, dans lequel l'huile respectueuse de la santé est l'huile de tournesol, l'huile de chanvre, l'huile de lin ou l'huile de sésame,
**caractérisé en ce que**
l'agent de soins dentaires contient un mélange constitué d'au moins deux huiles respectueuses de la santé et l'agent de soins dentaires contient 33,2 % en poids d'eau saline, dans lequel l'agent de soins dentaires contient entre 25 et 50 % en poids de l'huile respectueuse de la santé, dans lequel l'eau saline est une solution saline aqueuse, laquelle contient au moins 14 g de sel par kg d'eau, dans lequel l'eau saline est une solution de chlorure de sodium dans l'eau et la concentration de l'eau saline est supérieure à 15 % en poids.

2. Agent de soins dentaires selon l'une quelconque des revendications précédentes, **caractérisé en ce que** l'eau saline est une eau saline à base d'hydrolat.

3. Agent de soins dentaires selon l'une quelconque des revendications précédentes, **caractérisé en ce que** l'agent de soins dentaires contient entre 5 et 10 % en poids de craie.

4. Agent de soins dentaires selon l'une quelconque des revendications précédentes, **caractérisé en ce que** l'agent de soins dentaires contient entre 15 et 50 % en poids de terre médicinale.
